# FASCICULE DE BREVET EUROPEEN

(11) **EP 0 656 920 B1**
(45) Date de publication et mention de la délivrance du brevet: **16.06.1999**
(21) Numéro de dépôt: 94918912.0
(22) Date de dépôt: 10.06.1994
(51) Int. Cl.: C09B 61/00, C12P 17/06, C07D 311/60, A61K 7/00

(54) **COMPOSITION COSMETIQUE CONTENANT EN TANT QUE SUBSTANCE COLORANTE AU MOINS UN DERIVE DE LA 5-METHOXY 8-METHYL 2-PHENYL 7H-1-BENZOPYRAN-7-ONE**
KOSMETISCHE ZUSAMMENSETZUNG DIE ALS FARBSTOFF MINDESTENS EIN DERIVAT DER 5-METHOXY 8-METHYL 2-PHENYL 7H-1-BENZOPYRAN-7-ONE ENTHÄLT
COSMETIC COMPOSITION CONTAINING AT LEAST ONE DERIVATIVE OF 5-METHOXY 8-METHYL 2-PHENYL 7H-1-BENZOPYRAN-7-ONE AS A COLOURING

(30) Priorité: 10.06.1993 FR 9307002
(43) Date de publication de la demande: 14.06.1995
(73) Titulaire: L'OREAL, 75008 Paris (FR)
(72) Inventeur: BELCOUR, Béatrice, F-37000 Tours (FR); MARTIN, Richard, F-37290 Vouvray (FR); HUSSLER, Georges, F-93600 Aulnay-sous-Bois (FR)
(74) Mandataire: Tonnellier, Jean-Claude
(86) Numéro de dépôt international: FR9400695
(87) Numéro de publication internationale: WO9429388

(56) Documents cités:
- EP-A- 0 387 739
- JAPANESE PATENTS GAZETTE Week 8636, Derwent Publications Ltd., London, GB; AN 86-236264 & JP,A,61 166 396 (NITTO ELECTRIC) 28 Juillet 1986 cité dans la demande
- DATABASE WPI Week 8907, Derwent Publications Ltd., London, GB; AN 89-051783 & JP,A,64 002 593 (POKKA) 6 Janvier 1989
- CHEMISCHE BERICHTE vol. 77, no. 5 , 1 Mai 1944 , WEINHEIM (DE) pages 347 - 353 BROCKMANN ET AL 'Ueber Benzopyrylium Verbindungen' cité dans la demande
- SEIFEN/OELE/FETTE/WACHSE vol. 113, no. 10 , 25 Juin 1987 , AUGSBOURG.DE pages 335 - 336 J.M FIGUERAS ET AL 'Rote Farbpigmente für dekorative Kosmetika'
- DATABASE WPI Week 9042, Derwent Publications Ltd., London, GB; AN 90-315507 & JP,A,2 222 691 (NIPPON KAYAKU KK) 5 Septembre 1990
- Traduction du document JP-A-61 166 396

## Description

L'invention a pour objet une composition cosmétique contenant en tant que substance colorante au moins un dérivé de la 5-méthoxy 8-méthyl 2-phényl 7H-1-benzopyran-7-one.

Plus particulièrement, la présente invention a pour objet une composition cosmétique contenant en tant que substance colorante, un mélange de dérivés de la 5-méthoxy 8-méthyl 2-phényl 7H-1-benzopyran-7-one issus de cultures tissulaires de Basella rubra, dans un milieu approprié.

Il est bien connu que la plupart des compositions cosmétiques, qu'elles soient destinées au maquillage ou aux soins de la peau ou des cheveux, contiennent, à des concentrations variables, au moins une substance colorante, celle-ci pouvant être du type pigmentaire et étant destinée soit à colorer les ongles, les lèvres ou les cheveux, soit à impartir à la composition une certaine coloration favorisant son aspect visuel.

De nombreuses substances colorantes ont déjà été proposées en vue de leur incorporation dans des compositions cosmétiques telles que par exemple des rouges à lèvres, des vernis à ongles, des lotions capillaires, ainsi que dans des produits de maquillage pour le visage, sous forme de fonds de teint ou de crèmes plus ou moins fluides.

Jusqu'à présent, le choix des substances colorantes était fonction de la nature des compositions cosmétiques, ce qui constituait un sérieux inconvénient dans la mesure où leur sélection devait résulter de nombreux essais.

On a maintenant constaté de façon surprenante qu'il était possible d'utiliser indiféremment dans des compositions cosmétiques, une certaine classe de substances colorantes, essentiellement rouge à rouge fonçé qui sont des dérivés de la 5-méthoxy 8-méthyl 2-phényl 7H-1-benzopyran-7-one.

La présente invention a donc pour objet une composition cosmétique contenant, dans un véhicule cosmétique approprié, en tant que substance colorante, au moins un composé correspondant à la formule (I) suivante : 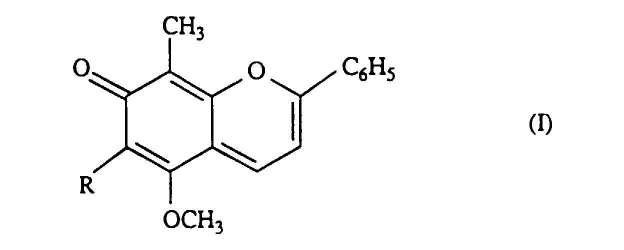 dans laquelle :
R représente un radical méthyle, hydroxyméthyle ou méthoxyméthyle.

Les composés de formule (I) sont plus particulièrement les suivants :
la 5-méthoxy 6,8-diméthyl 2-phényl 7H-1-benzopyran-7-one (composé Ia),
la 5-méthoxy 6-hydroxyméthyl 8-méthyl 2-phényl 7H-1-benzopyran-7-one (composé Ib), et
la 5-méthoxy 6-méthoxyméthyl 8-méthyl 2-phényl 7H-1-benzopyran-7-one (composé Ic).

Le composé Ia est un composé connu dont la préparation a été décrite par Borckmann et al, Chemische Brichte, vol. 77, N° 5, p. 347-353, 1944.

Les composés Ib et Ic sont nouveaux et peuvent être obtenus selon le même mode opératoire. Ces composés constituent également un des objets de l'invention.

Les composés de formule (I) et en particulier leurs mélanges peuvent être également obtenus par culture de cals de Basella rubra.

La présente invention a donc en outre pour objet un nouveau procédé de préparation de substances colorantes rouges à partir de cals obtenus par culture tissulaire de Basella rubra dans un milieu approprié.

Dans la demande JP 166 396, il a été décrit la préparation de substances colorantes rouges à partir de cals obtenus par culture tissulaire de Basella rubra en milieu supplémenté en auxine telle que l'acide 2,4-dichlorophénoxyacétique (2,4-D) et en cytokinine telle que la kinétine, suivie d'une étape de production de substances colorantes par culture desdits cals, dans l'obscurité, sur un milieu de culture contenant, en vue d'augmenter le rendement, une combinaison d'une auxine telle que l'acide naphtalène acétique (ANA) et d'une cytokinine telle que la kinétine ou la benzyladénine.

Les substances colorantes obtenues selon le procédé décrit dans cette demande sont des substances colorantes déjà présentes dans la plante de départ.

De façon surprenante et inattendue, on a pu constater que lorsque l'on effectuait la production de cals de Basella rubra dans un premier milieu de culture en présence d'acide naphtalène-acétique et d'une cytokinine et que l'on transférait et cultivait les cals obtenus dans un milieu nutritif contenant de l'acide 2,4-dichlorophénoxyacétique (2,4-D), il était possible d'obtenir dans ce second milieu et sans recourir à des opérations particulières des substances colorantes différentes de celles décrites dans la demande JP 166 396 et qui ne sont pas présentes dans le végétal de départ.

Ce résultat, particulièrement surprenant, est dû essentiellement au fait que par rapport à la demande JP 166 396, on utilise selon le procédé de l'invention dans le deuxième milieu de culture, non pas l'acide naphtalène-acétique mais l'acide 2,4-dichlorophénoxyacétique et que, par ailleurs, on utilise dans le premier milieu de culture de l'acide naphtalène-acétique associé à une cytokinine.

La présente invention a donc également pour objet un procédé de préparation d'au moins une substance colorante correspondant à la formule générale (I), ce procédé consistant à préparer et cultiver des cals de Basella rubra dans un premier milieu nutritif approprié contenant de l'acide naphtalène-acétique et une cytokinine, puis à transférer et à cultiver les cals obtenus dans un second milieu nutritif, appelé milieu de transfert, contenant une auxine, ladite auxine étant l'acide 2,4-dichlorophénoxyacétique, à séparer le milieu de transfert de la biomasse et à soumettre ledit milieu de transfert à une extraction et éventuellement à isoler l'un au moins des composés de formule (I) selon les méthodes connues.

Dans un mode de réalisation particulier du procédé selon l'invention, celui-ci peut encore présenter les caractéristiques suivantes, prises isolément ou éventuellement en combinaison :
- la cytokinine du premier milieu de culture est de préférence la kinétine ;
- le second milieu nutritif contient en outre une cytokinine, celle-ci étant notamnent la kinétine ;
- l'étape de préparation des cals est effectuée sous un éclairage suffisant pour obtenir des cals chlorophylliens, en revanche, l'étape de biosynthèse des colorants après transfert des cals dans le second milieu nutritif peut être effectuée soit dans l'obscurité, soit sous éclairement ;
- le premier et le second milieu nutritif sont du type Murashige-Skoog ; et
- les cals utilisés sont des cals de Basella rubra variété alba.

Comme mentionné ci-dessus, après un temps de culture suffisant, le milieu de transfert est séparé de la biomasse et on le soumet à une extraction à l'aide d'un solvant qui peut être choisi parmi le dichlorométhane, le méthanol, l'acétate d'éthyle et le chloroforme ou leurs mélanges. Lorsque l'on utilise un solvant miscible à l'eau, tel que le méthanol, on élimine d'abord l'eau puis on reprend le résidu par ce solvant.

L'extrait obtenu après concentration peut être éventuellement purifié, en vue d'isoler, selon des méthodes connues, par exemple par chromatographie sur couche mince ou sur colonne, les composés Ia,Ib et Ic tels que définis ci-dessus.

La présente invention a également pour objet un composé répondant à la formule générale (II) suivante : 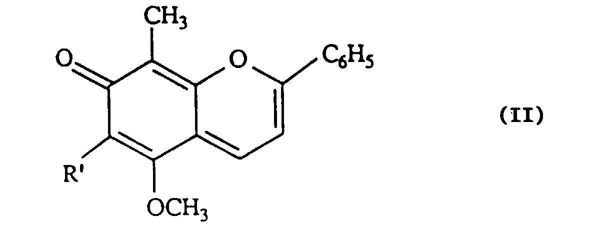 dans laquelle :
R' représente un radical hydroxyméthyle ou méthoxyméthyle.

Les composés de formule (II) sont plus particulièrement les suivants :
- la 5-méthoxy 6-hydroxyméthyl 8-méthyl 2-phényl 7H-1-benzopyran-7-one, et
- la 5-méthoxy 6-méthoxyméthyl 8-méthyl 2-phényl 7H-1-benzopyran-7-one.

Il va de soi que selon l'objet principal de l'invention, à savoir les compositions cosmétiques, la substance colorante, peut être sous forme d'un extrait, c'est-à-dire sous forme d'un mélange ou sous forme d'un composé individuel isolé selon le procédé tel que décrit ci-dessus ou obtenu par voie de synthèse organique.

Dans les compositions cosmétiques selon l'invention, la concentration en substance colorante telle que définie précédemment est généralement comprise entre 0,0001 à 20 % en poids par rapport au poids total de la composition.

Lorsque la substance colorante est plus particulièrement destinée à des produits de maquillage, tels que des rouges à lèvres, des vernis à ongles, des poudres ou encore des fonds de teint, la concentration est de préférence comprise entre 0,1 à 20 % en poids et plus particulièrement entre 0,5 à 10 % en poids par rapport au poids total de la composition.

Par contre, lorsque la substance colorante est uniquement destinée à donner une coloration à des compositions cosmétiques, telles que par exemple des crèmes, des gels huileux ou des masques pour le visage, la concentration est alors nettement plus faible et est de préférence comprise entre 0,0001 à 1 % en poids par rapport au poids total de la composition.

Lorsque les compositions selon l'invention sont destinées à un usage capillaire, en vue conférer aux cheveux une certaine coloration, la concentration en substance colorante est généralement comprise entre 0,05 et 10% en poids et de préférence entre 0,1 et 2,5% en poids par rapport au poids total de la composition.

Comme ceci résulte des pourcentages mentionnés ci-dessus, la concentration en substance colorante peut varier dans de larges limites et dépend essentiellement de l'intensité de la coloration que l'on souhaite conférer.

Selon l'invention, les compositions cosmétiques peuvent être liquides, semi-solides ou solides et se présenter sous diverses formes telles que par exemple sous forme de sticks, de pâtes, de crèmes, anhydres ou aqueuses, d'émulsions, de suspensions, de dispersions ou de solutions et constituer des rouges à lèvres, des mascaras, des fards à joues, des fards à paupières, des fonds de teint, des poudres, compactes ou non, ou encore des vernis à ongles.

Selon l'invention, la substance colorante telle que précédemment définie peut être associée à des pigments minéraux ou organiques, notamment à des laques, telles que les laques de calcium de D et C Red N° 7, les laques de baryum des D et C Red N° 6 et 9, les laques d'aluminium des D et C Red N° 3 et D et C Yellow N° 5 et les laques de zirconium de D et C Orange N° 5. A cette liste, on peut également inclure les D et C Red 30 et 36 qui, du fait de leur insolubilité dans l'eau et dans les huiles, sont généralement considérés comme des pigments bien qu'ils ne se présentent pas sous forme de laques métalliques.

Parmi les pigments inorganiques, on peut en particulier citer : les oxydes de fer (rouge, brun, noir et jaune), de chrome, les outremers (polysulfures d'aminosilicates), le dioxyde de titane, le pyrophosphate de manganèse et le bleu de prusse (ferrocyanure ferrique). Ces différents composés seuls ou en mélange sont généralement employés à une concentration comprise entre 0,1 et 40 % par rapport au poids total de la composition.

Par ailleurs, ces compositions peuvent également contenir des agents nacrants tels que l'oxychlorure de bismuth, le micatitane et les cristaux de guanine.

Lorsque les compositions se présentent sous forme de sticks, notamment de rouges à lèvres, de fards à paupières ou à joues et de fonds de teint, une partie importante de ces compositions est constituée par le corps gras qui peut être au moins une cire telle que par exemple l'ozokérite, la lanoline, l'alcool de lanoline, la lanoline hydrogénée, la lanoline acétylée, la cire de lanoline, la cire d'abeille, la cire de Candellila, la cire microcristalline, la cire de carnauba, l'alcool cétylique, l'alcool stéarylique, le beurre de cacao, les acides gras de lanoline, le petrolatum, les vaselines, les mono, di et tri-glycérides concrets à 25 % C, les esters gras concrets à 25 % C, les cirés de silicone telles que le méthyloctadécanoxypolysiloxane et le poly(diméthylsiloxy)stéaroxysiloxane, le monoéthanolamide stéarique, la colophane et ses dérivés tels que les abiétates de glycol et de glycérol, les huiles hydrogénées concrets à 25 % C, les sucroglycérides et les oléates, myristates, lanolates, stéarates et dihydroxystearates de calcium, magnesium, zirconium et d'aluminium.

Le corps gras peut également être constitué d'un mélange d'au moins une cire et d'au moins une huile et dans ce cas, l'huile peut être par exemple : l'huile de paraffine, l'huile de purcellin, le perhydrosqualène, l'huile d'amande douce, l'huile d'avocat, l'huile de calophyllum, l'huile de ricin, l'huile de sésame, l'huile de jojoba, les huiles minérales ayant un point d'ébullition compris entre 310 et 410°C, les huiles de silicone telles que les diméthylpolysiloxanes, l'alcool linoléïque, l'alcool linolénique, l'alcool oléïque, l'huile de germes de blé, le lanolate d'isopropyle, le palmitate d'isopropyle, le myristate d'isopropyle, le myristate de méthyle, le myristate de céthyle, le stéarate d"hexadécyle, le stéarate de butyle, l'oléate de décyle, les acétyl-glycérides, les octanoates et décanoates d'alcools et de polyalcools tels que ceux de glycol et de glycérol, les ricinoléates d'alcools et de polyalcools tels que le ricinoléate de cétyle, l'alcool isostéarylique, le lanolate d'isocétyle, l'adipate d'isopropyle, le laurate d'héxyle et l'octyldodécanol.

De façon générale, le corps gras dans ces compositions sous forme de stick peut représenter jusqu'à 99,9% en poids du poids total de la composition.

Ces compositions cosmétiques peuvent également contenir d'autres ingrédients, tels que par exemple des glycols, des polyéthylèneglycols, des polypropylèneglycols, des monoalcanolamides, des polymères non colorés, des charges minérales ou organiques, des conservateurs, des filtres U.V. ou d'autres additifs usuels en cosmétique.

Ces compositions sous forme de stick sont de préférence anhydres. Toutefois, dans certains cas, elles peuvent contenir une certaine quantité d'eau, n'excédant pas généralement 40% par rapport au poids total du stick.

Quand les compositions cosmétiques selon l'invention se présentent sous forme semi-solide, c'est-à-dire sous forme de pâtes ou de crèmes, elles peuvent être soit anhydres soit aqueuses et constituent des fonds de teint, des fards à joues, des fards à paupières, des rouges à lèvres, etc ...

Lorsque ces pâtes ou crèmes sont par contre aqueuses, il s'agit alors plus particulièrement d'émulsions du type eau-dans-l'huile ou huile-dans-l'eau dont la phase grasse représente de 1 à 98,8% en poids, la phase eau de 1 à 98,8% et l'agent émulsionnant de 0,1 à 30% en poids.

Ces compositions peuvent également contenir d'autres ingrédients conventionnels tels que des parfums, des agents anti-oxydants, des conservateurs, des gélifiants, des filtres U.V., des colorants, des pigments, des agents nacrants, des polymères non colorés et des charges minérales ou organiques.

Lorsque les compositions cosmétiques selon l'invention se présentent sous forme d'une poudre, elles sont essentiellement constituées par une charge minérale ou organique telles que du talc, du kaolin, des amidons, des poudres de polyéthylène ou des poudres de polyamides ainsi que des additifs tels que des liants, des colorants, etc ...

De telles compositions peuvent également contenir divers additifs habituels en cosmétique tels que des parfums, des anti-oxydants, des agents conservateurs, etc ....

Lorsque les compositions selon l'invention se présentent sous forme de vernis à ongles, elles sont essentiellement constituées de nicrocellulose et d'un polymère naturel ou synthétique, en solution dans un système solvant, cette solution contenant éventuellement d'autres additifs tels que des pigments et/ou des agents nacrants.

Lorsque les compositions selon l'invention sont destinées à un usage capillaire, celles-ci peuvent se présenter sous forme de lotions, d'émulsions ou encore de lotions épaissies ou de gels.

Lorsque les compositions capillaires se présentent sous forme de lotions, celles-ci comprennent généralement en solution aqueuse, alcoolique ou hydroalcoolique, la substance colorante ainsi que divers additifs conventionnels dans ce type de composition.

Ces compositions peuvent éventuellement se présenter sous forme aérosol comprenant un gaz propulseur tel que le gaz carbonique, l'azote, le protoxyde d'azote, les hydrocarbures volatils tels que le butane, l'isobutane ou le propane ou encore des hydrocarbures halogénés.

Lorsque les compositions se présentent sous forme d'émulsions, celles-ci peuvent être non ioniques ou anioniques. Les émulsions non ioniques sont constituées principalement d'un mélange d'huile et/ou d'alcool gras, et d'alcool polyéthoxylé tels que les alcools stéarylique ou cétéarylique, polyéthoxylés. On peut éventuellement ajouter à ces compositions des tensio-actifs cationiques.

Les émulsions anioniques sont consituées essentiellement à partir de savons.

Lorsque les compositions se présentent sous forme de lotions épaissies ou encore de gels, elles contiennent des épaississants en présence ou non de solvants. Les épaississants utilisables peuvent être l'alginate de sodium, la gomme arabique ou la gomme de xanthane ou des dérivés cellulosiques tels que la méthylcellulose, l'hydroxyméthylcellulose, l'hydroxyéthylcellulose, l'hydroxypropylcellulose, l'hydroxypropylméthylcellulose, la carboxyméthylcellulose. On peut également obtenir un épaississement des lotions par mélange de polyéthylèneglycol et de stéarate ou de distéarate de polyéthylèneglycol ou par un mélange d'ester phosphorique et d'amide. La concentration en épaississant peut varier de 0,5 à 30% en poids et avantageusement de 0,5 à 15% en poids et de préférence de 0,5 à 5%. Le pH des lotions varie essentiellement entre 3 et 9 et de préférence entre 4,5 et 7,5.

On va maintenant donner à titre d'illustration un exemple de préparation des substances colorantes de formule (I) ainsi que plusieurs exemples de compositions cosmétiques selon l'invention.

### EXEMPLE 1

### a) La plante utilisée est Basella rubra, variété alba, à feuilles et tiges vertes.

On prélève des explants de tiges et/ou de pétioles foliaires ayant environ 0,5 cm de long.

Les explants sont désinfectés par immersion dans une solution de chlorure mercurique à 1 g/l pendant une minute et 30 secondes puis sont soumis à trois rinçages successifs à l'eau stérile afin d'éliminer les résidus de chlorure mercurique.

Les explants sont placés, dans des conditions d'aseptie, dans des boîtes de culture contenant un milieu de culture A gélosé constitué essentiellement d'un milieu nutritif de type Murashige-Skoog supplémenté en acide naphtalène-acétique (1 mg/l) et en kinétine (1 mg/l).

Les conditions de culture sont les suivantes :
- conditions d'éclairement : 5 µ Einstein/m²/s ;
- photopériode : 16 h ;
- température de culture : 26°c.

Les boîtes de culture sont des boîtes de Pétri de 45 mm de diamètre contenant 12 ml de milieu de culture par boîte.

Au bout d'un mois, on obtient des cals primaires qui sont de type chlorophyllien et qui présentent des émergences racinaires.

Ces cultures de cals peuvent être entretenues par repiquage mensuel.

### b) Transfert en milieu liquide

Des tissus végétaux obtenus après sept cycles de repiquage après culture selon le procédé a) ci-dessus sont transférés en milieu liquide. Pour cela, 2 g de tissus obtenus après 1 mois de culture comme décrit en a) sont transférés dans une fiole d'Erlenmeyer de 100 ml contenant 50 ml de milieu A' (milieu A sans agar). Les fioles sont placées en agitation à 100 rpm, à 26°C, sous un éclairement de 5µ Einstein/m²/s (photopériode de 12 heures). Après 8 jours de culture dans ces conditions, on observe l'apparition et le développement de structures de type globulaire.

Ces tissus peuvent être entretenus par repiquage périodique, tous les 15 jours par exemple, après filtration et inoculation dans un milieu A' neuf.

### c) Induction de la formation de métabolites colorés

Des tissus en culture sur milieu A' et âgés de 15 jours sont filtrés sur toile de Blutex 50 µm puis sont répartis stérilement en fioles d'Erlenmeyer de 250 ml contenant 100 ml de milieu nutritif B, l'inoculation étant faite à raison de 25 g/l.

Le milieu B est constitué d'un milieu nutritif de type Murashige-Skoog supplémenté en 2,4-D (1 mg/l) et en kinétine (1 mg/l).

Les conditions de culture sont les suivantes :
- agitation à 100 rpm ;
- éclairement : 5 micro Einstein par m²/s ;
- photopériode : 12 heures ;
- température : 26°C.

A l'issue d'une période de latence de 24-48 heures, on observe l'apparition d'une coloration rose dans le milieu de culture.

La recherche des métabolites colorés dans les tissus et dans le milieu de culture à différents stades de la biosynthèse montre que ces métabolites ne sont pas stockés par les cellules mais diffusent dans le milieu.

La biosynthèse des métabolites s'accompagne d'une absence de croissance des tissus.

Pour assurer une continuité de la production, on peut procéder au renouvellement du milieu de culture. Pour cela le milieu de culture contenant les métabolites colorés est séparé par filtration sur toile de Blutex de 50 µm. Les tissus recueillis sont repris et transférés dans les mêmes conditions d'inoculation et de culture dans un milieu B neuf et stérile.

On a procédé en outre à des cultures analogues, dans des milieux analogues au milieu B, mais supplémentés à différentes concentrations de 2,4-D (2,5 ; 5 ; 7,5 et 10 mg/l).

On a constaté que la production de métabolites colorés augmente avec la teneur en 2,4-D dans le milieu.

### Influence du renouvellement du milieu

Le milieu de production peut être renouvelé. Pour cela, on filtre la culture, par exemple sur toile de Blutex de 50 µm et les tissus végétaux sont transférés dans les mêmes conditions d'inoculation et de culture dans un milieu B neuf supplémenté. On a constaté que, dans ces conditions, la productivité, après renouvellement du milieu, est augmentée d'un facteur 1,4.

### Influence de l'éclairement

La production des métabolites colorés peut être effectuée sous éclairement ou à l'obscurité. A l'obscurité, le rendement peut être doublé, mais les proportions respectives des composés Ib et Ia sont différentes selon les conditions d'éclairement.

### Extraction des composés

Les milieux de culture séparés par filtration comme décrit précédemment sont acidifiés par l'acide chlorhydrique 1N jusqu'à un pH de 2,5. On procède ensuite à deux extractions successives par un volume de dichlorométane avec agitation pendant une heure à chaque fois.

Dans le cas de milieux de culture gélosés, on procède à trois extractions successives par un volume de méthanol.

Les extraits organiques obtenus sont ensuite concentrés à sec sous pression réduite à 50°C, remis en solution dans le méthanol puis analysés en chromatographie en couche mince sur plaque de silice (origine : Merck Kieselgel 60 F2-54) en utilisant comme éluant un mélange dichlorométhane-méthanol 95:5 (v/v). On distingue 3 composés caractérisés respectivement par des Rf de 0,45, de 0,40 et de 0,30.

L'analyse ultérieure des composés a montré que le composé de Rf 0,45 est le composé Ia, le composé de Rf 0,30 est le composé Ib et le composé de Rf 0,40 est le composé Ic.

Les composés Ia et Ib ont été isolés par une chromatographie à moyenne pression sur une colonne de silice en utilisant le mélange dichlorométhane-méthanol 94:6 comme éluant. Cette séparation a permis de collecter en outre une fraction enrichie en composé Ic qui a ensuite été purifiée par chromatographie sur couche mince (silice, éluant : acétate d'éthyle-dichlorométhane-heptane 70:20:10).

La chromatographie à moyenne pression a été effectuée avec de la silice commercialisée sous la dénomination Merck Kieselgel (40-63 µm).

La chromatographie à moyenne pression de 400 mg d'extraits, en collectant des fractions de 25 ml, a été effectuée en utilisant un mélange éluant de dichlorométhane-méthanol 96:4. Pour un extrait obtenu sur une culture âgée de 14 à 16 jours sur milieu B, on a obtenu 52 mg de composé Ia, 41 mg de composé Ib et 18 mg d'un mélange enrichi en composé Ic. Par chromatographie sur couche mince, en utilisant comme éluant un mélange acétate d'éthyle-dichlorométhane-heptane 70:20:10 puis un mélange dichlorométhane-méthanol 93:7, on a obtenu 1,3 mg de composé Ic.

Compte-tenu de ce fractionnement sur colonne, l'extrait obtenu précédemment contient environ :
- 13% du composé Ia
- 10,2% du composé Ib
- 0,3% du composé Ic.

La structure des composés Ia, Ib et Ic a été établie par analyses de spectrométrie de masse, de spectroscopie U.V., IR, RMN¹H,¹³C, et par une combinaison de RMN²D en corrélation hétéronucléaire ¹H¹³C et longue distance en détection inverse.

Les spectres infrarouges ont été enregistrés par dépôt et évaporation d'une solution des composés Ia, Ib et Ic dans le chloroforme sur une fenêtre de NaCl à l'aide d'un appareil Brucker IFS 85.

Les spectres UV ont été enregistrés dans le chloroforme sur un appareil Shimadzu UV 2101 PC.

Les spectres de masse ont été obtenus en EI à l'aide d'un spectromètre de type quadrapolaire Incos 50 (Finnigan).

Les spectres de RMN¹H¹³C et ²D ont été effectués sur un appareil Brucker AMX 500 MHz en utilisant le chloroforme deutérié comme référence interne.

Ces analyses ont permis d'établir la structure qui est celle indiquée ci-dessus.

A titre d'exemple le spectre d'absorption (UV) dans l'éthanol présente les maxima suivantes :
- composé Ia : 495, 375, 326 et 255 nm.
- composé Ib : 503, 365, 325, 275 et 244 nm

Des essais de coloration directe à pH spontané sur mèches de cheveux se sont avérés positifs. Le pouvoir colorant des composés de l'invention a également été observé sur différents substrats tels que des toiles de nylon, des polymères de silicone, etc.

### Stabilité de la coloration

### a) Stabilité à la lumière et à la chaleur :

Des essais ont été effectués sur des extraits bruts par exposition à la lumière à 254 nm pendant 6 heures ou à la chaleur par maintien pendant 6 heures à une température de 50 ou de 80°C. On n'observe aucune modification de couleur.

De plus, les colorations obtenues sur substrats présentent une bonne résistance aux lavages.

Avec un traitement analogue mais à 100°C la couleur initiale rouge-rosée de l'extrait témoin prend une teinte modifiée rouge-orangée.

### b) Stabilité au pH :

L'extrait brut en phase aqueuse à pH compris entre 3,5 et 10,5 ne montre aucune variation de couleur (rouge-rosée).

A pH inférieur à 3,5 et à pH supérieur à 10,5, la couleur initiale rouge-rosée prend une teinte modifiée rouge-orangée.

### EXEMPLE 2

Composition colorante capillaire sous forme de lotion contenant les ingrédients suivants :
- Extrait de culture de cals de Basella rubra, obtenu selon l'exemple 1 0,80g
- Ethanol à 96 % (vol/vol) 30,00g
- Triethanolamine qs pH = 8
- Eau qsp 100,00g

Une mèche de 0,15 g de cheveux gris permanentés (à 90 % de cheveux blancs) est immergée dans 10 g de la composition colorante ci-dessus pendant 30 minutes.

Après rinçage et séchage, les cheveux sont colorés en une nuance rose.

### EXEMPLE 3

Composition colorante capillaire sous forme de lotion contenant les ingrédients suivants :
- Extrait de culture de cals de Basella rubra, obtenu selon l'exemple 1 0,23g
- Copolymère vinyl pyrrolidone/acétate de vinyle (65/35) 1,50g
- Ethanol à 96 % (vol/vol) 30,00g
- 2-amino 2-méthyl 1-propanol qs pH = 9
- Eau qsp 100,0 g

On applique 0,15 g de cette composition colorante pour 1 g de cheveux gris naturels (à 90 % de cheveux blancs). Après un temps de pause de 30 minutes et séchage, les cheveux sont colorés en une nuance blond cendré très clair.

### EXEMPLE 4

Composition cosmétique sous forme de rouge à lèvres contenant les ingrédients suivants :
- Lanoline liquide 19,50g
- Cire microcristalline 15,00g
- Triglycérides des acides caprique/caprylique vendus sous la dénomination de "Miglyol 812" par la Société Hüls AG 11,00g
- Béhénate d'octyl glycéryle 11,00g
- Huile de sésame 10,00g
- Oxyde de titane 3,00g
- Extrait de culture de cals de Basella Rubra obtenu selon l'exemple 1 3,00g
- Butyl hydroxytoluène 0,20g
- Huile de ricin qsp 100,00g

### EXEMPLE 5

Composition cosmétique sous forme d'un fard à joues contenant :
- Huile de vaseline 6,00g
- Micatitane 10,00g
- Dioxide de titane 10,00g
- Oxyde de fer rouge 0,90g
- Oxyde de fer noir 0,10g
- Extrait de culture de cals de Basella rubra obtenu selon l'exemple 1 1,20g
- Talc qsp 100,00g

### EXEMPLE 6

Composition cosmétique sous forme d'une poudre compacte pour le visage contenant :
- Mica 10,00g
- Poudre de nylon 10,00g
- Dioxyde de titane 10,00g
- Myristate d'isopropyle 1,50g
- Huile de vaseline 1,50g
- Extrait de culture de cals de Basella rubra obtenu selon l'exemple 1 0,50g
- Oxydes de fer 3,00g
- Talc qsp 100,00g

### EXEMPLE 7

Composition cosmétique sous forme d'une crème de jour contenant :
- Stéarate de glycérol autoémulsionnable vendu sous la dénomination de "ARLACEL 165" par la Société ICI 3,00 g
- Alcool cétéarylique 1,50 g
- Triéthanolamine 0,40 g
- Homopolymère de l'acide acrylique réticulé (CARBOMER) 0,40 g
- Huile d'abricot 12,00 g
- Polyisobutène 6,00 g
- Pyrrolidone carboxylate de sodium 3,00 g
- Cyclopentadiméthylsiloxane 5,00 g
- Imidazolinyl urée vendue sous la dénomination de "GERMAL 115" par la Société SUTTON 0,20 g
- Extrait de culture de cals de Basella rubra obtenu selon l'exemple 1 0,005g
- Oxyde de fer jaune 0,001g
- Conservateurs 0,30 g
- Eau qsp 100,00 g

## Revendications

1. Composition cosmétique caractérisée par le fait qu'elle contient, dans un véhicule cosmétique approprié, en tant que substance colorante, au moins un composé répondant à la formule suivante : 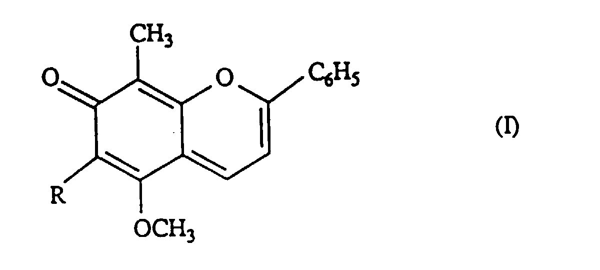 dans laquelle :
R représente un radical méthyle, hydroxyméthyle ou méthoxyméthyle.

2. Composition selon la revendication 1 caractérisée par le fait que ladite substance colorante est choisie parmi:
- la 5-méthoxy 6,8-diméthyl 2-phényl 7H-1-benzopyran-7-one (composé Ia),
- la 5-méthoxy 6-hydroxyméthyl 8-méthyl 2-phényl 7H-1-benzopyran-7-one (composé Ib),
- la 5-méthoxy 6-méthoxyméthyl 8-méthyl 2-phényl 7H-1-benzopyran-7-one (composé Ic),
et leurs mélanges.

3. Composition selon l'une quelconque des revendications précédentes caractérisée par le fait qu'elle se présente sous forme liquide, semi-solide ou solide.

4. Composition selon l'une quelconque des revendications 1 à 3 caractérisée par le fait qu'elle contient ladite substance colorante en une proportion comprise entre 0,0001 et 20 % en poids par rapport au poids total de la composition.

5. Composition selon l'une quelconque des revendications 1 à 4 caractérisée par le fait qu'elle se présente sous forme de stick contenant de 0,1 à 20 % en poids de substance colorante et jusqu'à 99,9 % en poids d'un corps gras constitué d'au moins une cire et éventuellement d'au moins une huile.

6. Composition selon la revendication 5 caractérisée par le fait qu'elle contient jusqu'à 40 % en poids d'eau par rapport au poids total de la composition.

7. Composition selon l'une quelconque des revendications 1 à 4 caractérisée par le fait qu'elle se présente sous forme de pâte ou de crème anhydre ou aqueuse.

8. Composition selon l'une quelconque des revendications 1 à 4 caractérisée par le fait qu'elle se présente sous forme d'une émulsion eau-dans-l'huile ou huile-dans-l'eau, la phase grasse représentant de 1 à 98,8 % en poids, la phase eau de 1 à 98,8 % et l'agent émulsionnant de 0,1 à 30 % en poids par rapport au poids total de la composition.

9. Composition selon l'une quelconque des revendications 1 à 4 caractérisée par le fait qu'elle se présente sous forme d'une poudre compacte et contient une charge choisie parmi le talc, le kaolin, l'amidon, la poudre de polyéthylène ou la poudre de polyamide.

10. Composition selon l'une quelconque des revendications 1 à 4 caractérisée par le fait qu'elle se présente sous forme d'un vernis à ongles contenant, dans une base pour vernis, de 0,1 à 20 % en poids de substance colorante.

11. Composition selon l'une quelconque des revendications 1 à 4 caractérisée par le fait qu'elle se présente sous forme d'une lotion capillaire aqueuse, alcoolique ou hydroalcoolique et contient ladite substance colorante en une proportion comprise entre 0,05 et 10 % en poids et de préférence entre 0,1 et 2,5 % en poids par rapport au poids total de la composition.

12. Procédé de préparation d'une substance colorante telle que définie aux revendications 1 et 2 caractérisé par le fait qu'il consiste à préparer et à cultiver des cals de Basella rubra, dans un premier milieu nutritif approprié contenant en tant qu'auxine de l'acide naphtalène-acétique et une cytokinine, puis à transférer et à cultiver les cals obtenus dans un second milieu nutritif, appelé milieu de transfert, contenant en tant qu'auxine de l'acide 2,4-dichlorophénoxyacétique, à séparer le milieu de transfert de la biomasse, à soumettre ledit milieu de transfert à une extraction et éventuellement à isoler les composés de formule (I) de l'extrait.

13. Procédé selon la revendication 12 caractérisé par le fait que ledit milieu de transfert contient en outre une cytokinine.

14. Procédé selon la revendication 12 ou 13 caractérisé par le fait que ladite cytokinine est la kinétine.

15. Procédé selon l'une quelconque des revendications 12 à 14 caractérisé par le fait que la préparation desdits cals est effectuée sous un éclairage suffisant pour obtenir des cals chlorophylliens.

16. Procédé selon l'une quelconque des revendications 12 à 15 caractérisé par le fait que ledit premier et ledit second milieu nutritif est de type Murashige-Skoog.

17. Procédé selon l'une quelconque des revendications 12 à 16 caractérisé par le fait que lesdits cals sont issus de Basella rubra, variété alba.

18. Procédé selon la revendication 12 caractérisé par le fait que l'extraction est réalisée à l'aide d'au moins un solvant choisi parmi le dichlorométhane, le méthanol, l'acétate d'éthyle et le chloroforme.

19. La 5-méthoxy 6-hydroxyméthyl 8-méthyl 2-phényl 7H-1-benzopyran 7-one.

20. La 5-méthoxy 6-méthoxyméthyl 8-méthyl 2-phényl 7H-1-benzopyran 7-one.

## Patentansprüche

1. Kosmetische Zubereitung, dadurch gekennzeichnet, daß sie in einem geeigneten kosmetischen Träger als farbgebende Substanz mindestens eine Verbindung entsprechend der folgenden Formel: 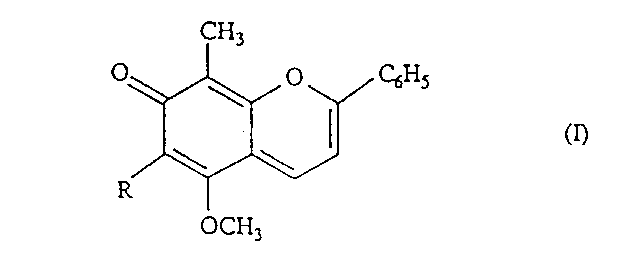 enthält, worin:
R für einen Methyl-, Hydroxymethyl- oder Methoxymethylrest steht.

2. Zubereitung gemäß Anspruch 1, dadurch gekennzeichnet, daß die farbgebende Substanz ausgewählt ist unter:
- dem 5-Methoxy-6, 8-dimethyl-2-phenyl-7H-1-benzopyran-7-on (Verbindung Ia),
- dem 5-Methoxy-6-hydroxymethyl-8-methyl-2-phenyl-7H-1-benzopyran-7-on (Verbindung Ib),
- dem 5-Methoxy-6-methoxymethyl-8-methyl-2-phenyl-7H-1-benzopyran-7-on (Verbindung lc),
sowie deren Mischungen.

3. Zubereitung gemäß einem der vorstehenden Ansprüche, dadurch gekennzeichnet, daß sie in flüssiger, halbfester oder fester Form vorliegt.

4. Zubereitung gemäß einem der Ansprüche 1 bis 3, dadurch gekennzeichnet, daß sie die besagte farbgebende Substanz in einer Menge zwischen 0,0001 und 20 Gew.-%, bezogen auf das Gesamtgewicht der Zubereitung, enthält.

5. Zubereitung gemäß einem der Ansprüche 1 bis 4, dadurch gekennzeichnet, daß sie in Form eines Sticks vorliegt, der 0,1 bis 20 Gew.-% der farbgebenden Substanz und bis zu 99,9 Gew.-% eines Fettkörpers enthält, der aus mindestens einem Wachs und gegebenenfalls mindestens einem Öl besteht.

6. Zubereitung gemäß Anspruch 5, dadurch gekennzeichnet, daß sie bis zu 40 Gew.-% Wasser, bezogen auf das Gesamtgewicht der Zubereitung, enthält.

7. Zubereitung gemäß einem der Ansprüche 1 bis 4, dadurch gekennzeichnet, daß sie in Form einer wasserfreien oder wäßrigen Paste oder Creme vorliegt.

8. Zubereitung gemäß einem der Ansprüche 1 bis 4, dadurch gekennzeichnet, daß sie in Form einer Wasser-in-Öl- oder Öl-in-Wasser-Emulsion vorliegt, wobei die Fettphase 1 bis 98,8 Gew.-%, die wäßrige Phase 1 bis 98,8 % und der Emulgator 0,1 bis 30 Gew.-%, bezogen auf das Gesamtgewicht der Zubereitung ausmacht.

9. Zubereitung gemäß einem der Ansprüche 1 bis 4, dadurch gekennzeichnet, daß sie in Form eines Kompaktpuders vorliegt und einen Zuschlag enthält, der ausgewählt ist unter Talkum, Kaolin, Stärke, Polyethylen- oder Polyamidpuder.

10. Zubereitung gemäß einem der Ansprüche 1 bis 4, dadurch gekennzeichnet, daß sie in Form eines Nagellacks vorliegt, der in einer Grundlage für Lacke 0,1 bis 20 Gew.-% der farbgebenden Substanz enthält.

11. Zubereitung gemäß einem der Ansprüche 1 bis 4, dadurch gekennzeichnet, daß sie in Form einer wäßrigen, alkoholischen oder hydroalkoholischen Lösung für die Haare vorliegt und die besagte farbgebende Substanz in einer Menge zwischen 0,05 und 10 Gew.-%, vorzugsweise zwischen 0,1 und 2,5 Gew.-%, enthält, bezogen auf das Gesamtgewicht der Zubereitung.

12. Verfahren zur Herstellung einer farbgebenden Substanz wie in den Ansprüchen 1 und 2 definiert, dadurch gekennzeichnet, daß es aus der Herstellung und Kultivierung von Kalli von *Basella rubra,* in einem ersten geeigneten Nährmedium, welches als Auxin Naphthalinessigsäure und ein Cytokinin enthält, anschließendem Transfer und Kultivierung der erhaltenen Kalli in einem zweiten Nährmedium, das als Transfermedium bezeichnet wird und als Auxin 2,4-Dichlorphenoxyessigsäure enthält, der Abtrennung des Transfermediums von der Biomasse, dem Unterwerfen des Transfermediums unter eine Extraktion und gegebenenfalls der Isolierung der Verbindungen der Formel (I) aus dem Extrakt besteht.

13. Verfahren gemäß Anspruch 12, dadurch gekennzeichnet, daß das Transfermedium zusätzlich ein Cytokinin enthält.

14. Verfahren gemäß Anspruch 12 oder 13, dadurch gekennzeichnet, daß es sich bei dem Cytokinin um das Kinetin handelt.

15. Verfahren gemäß einem der Ansprüche 12 bis 14, dadurch gekennzeichnet, daß die Herstellung der Kalli unter ausreichender Beleuchtung erfolgt, um chlorophyllhaltige Calli zu erhalten.

16. Verfahren gemäß einem der Ansprüche 12 bis 15, dadurch gekennzeichnet, daß das erste und zweite Nährmedium vom Typ Murashige-Skoog sind.

17. Verfahren gemäß einem der Ansprüche 12 bis 16, dadurch gekennzeichnet, daß die Kalli von *Basella rubra,* var. *alba* abstammen.

18. Verfahren gemäß Anspruch 12, dadurch gekennzeichnet, daß die Extraktion mit Hilfe mindestens eines Lösungsmittels erfolgt, ausgewählt unter Dichlormethan, Methanol, Ethylacetat und Chloroform.

19. 5-Methoxy-6-hydroxymethyl-8-methyl-2-phenyl-7H-1-benzopyran-7-on.

20. 5-Methoxy-6-methoxymethyl-8-methyl-2-phenyl-7H-1-benzopyran-7-on.

## Claims

1. Cosmetic composition, characterized in that it contains as colorant, in a suitable cosmetic excipient, at least one compound corresponding to the following formula: 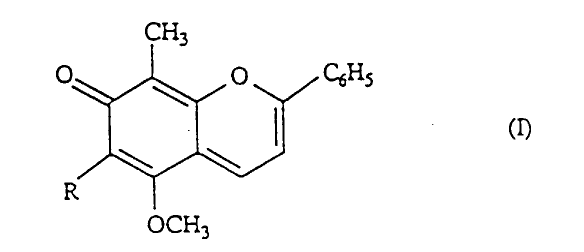 in which:
R represents a methyl, hydroxymethyl or methoxymethyl radical.

2. Composition according to Claim 1, characterized in that the said colorant is chosen from
- 5-methoxy-6,8-dimethyl-2-phenyl-7H-1-benzopyran-7-one (compound Ia),
- 5-methoxy-6-hydroxymethyl-8-methyl-2-phenyl-7H-1-benzopyran-7-one (compound Ib),
- 5-methoxy-6-methoxymethyl-8-methyl-2-phenyl-7H-1-benzopyran-7-one (compound Ic),
and mixtures thereof.

3. Composition according to either of the preceding claims, characterized in that it is presented in liquid, semi-solid or solid form.

4. Composition according to any one of Claims 1 to 3, characterized in that it contains the said colorant in a proportion of between 0.0001 and 20% by weight relative to the total weight of the composition.

5. Composition according to any one of Claims 1 to 4, characterized in that it is presented in the form of a stick containing from 0.1 to 20% by weight of colorant and up to 99.9% by weight of a fatty substance consisting of at least one wax and, if desired, at least one oil.

6. Composition according to Claim 5,characterized in that it contains up to 40% by weight of water relative to the total weight of the composition.

7. Composition according to any one of Claims 1 to 4, characterized in that it is presented in the form of a paste or an anhydrous or aqueous cream.

8. Composition according to any one of Claims 1 to 4, characterized in that it is presented in the form of a water-in-oil or oil-in-water emulsion, the fatty phase representing from 1 to 98.8% by weight, the water phase from 1 to 98.8% by weight and the emulsifier from 0.1 to 30% by weight relative to the total weight of the composition.

9. Composition according to any one of Claims 1 to 4, characterized in that it is presented in the form of a pressed powder and contains a filler chosen from talc, kaolin, starch, polyethylene powder and polyamide powder.

10. Composition according to any one of Claims 1 to 4, characterized in that it is presented in the form of a nail varnish containing, in a varnish base, from 0.1 to 20% by weight of colorant.

11. Composition according to any one of Claims 1 to 4, characterized in that it is presented in the form of an aqueous, alcoholic or aqueous-alcoholic hair lotion and contains the said colorant in a proportion of between 0.05 and 10% by weight and preferably between 0.1 and 2.5% by weight relative to the total weight of the composition.

12. Process for the preparation of a colorant as defined in Claims 1 and 2, characterized in that it consists in preparing and culturing calluses of Basella rubra in a first, suitable nutrient medium containing as auxin naphthaleneacetic acid and a cytokinin, then in transferring the calluses obtained to a second nutrient medium, referred to as the transfer medium, and culturing them therein, the transfer medium containing as auxin 2,4-dichlorophenoxyacetic acid, in separating the transfer medium from the biomass, in subjecting the said transfer medium to an extraction and, optionally, in isolating the compounds of formula (I) from the extract.

13. Process according to Claim 12, characterized in that the said transfer medium additionally contains a cytokinin.

14. Process according to Claim 12 or 13, characterized in that the said cytokinin is kinetin.

15. Process according to any one of Claims 12 to 14, characterized in that the preparation of the said calluses is carried out under lighting which is sufficient to obtain chlorophyllian calluses.

16. Process according to any one of Claims 12 to 15, characterized in that the said first and the said second nutrient medium is of the Murashige and Skoog type.

17. Process according to any one of Claims 12 to 16, characterized in that the said calluses are obtained from Basella rubra, variety alba.

18. Process according to Claim 12, characterized in that the extraction is carried out using at least one solvent chosen from dichloromethane, methanol, ethyl acetate and chloroform.

19. 5-methoxy-6-hydroxymethyl-8-methyl-2-phenyl-7H-1-benzopyran-7-one.

20. 5-methoxy-6-methoxymethyl-8-methyl-2-phenyl-7H-1-benzo-pyran-7-one.
